**Europäisches Patentamt**

**European Patent Office**

**Office européen des brevets**

(19)

(11) Numéro de publication : **0 417 018 B1**

(12)

# FASCICULE DE BREVET EUROPEEN

(45) Date de publication du fascicule du brevet :
**11.05.94 Bulletin 94/19**

(21) Numéro de dépôt : **90420393.2**

(22) Date de dépôt : **04.09.90**

(51) Int. Cl.⁵ : **C03C 3/112,** C03C 4/00,
C03C 10/16, C03C 14/00,
A61L 27/00, A61C 13/083,
A61K 6/027

(54) **Matériau bioréactif pour prothèse ou implants composites.**

(30) Priorité : **06.09.89 FR 8911857**

(43) Date de publication de la demande :
**13.03.91 Bulletin 91/11**

(45) Mention de la délivrance du brevet :
**11.05.94 Bulletin 94/19**

(84) Etats contractants désignés :
**AT CH DE DK ES FR GB GR IT LI LU NL SE**

(56) Documents cités :
**EP-A- 0 076 692
EP-A- 0 112 319
EP-A- 0 206 726
WO-A-87/06842
US-A- 4 478 904
US-A- 4 775 646
JOURNAL OF MATERIALS SCIENCE. vol. 23,
no. 12, décembre 1988, LONDON GB pages
4295 - 4299; FENG-HUEI LIN ET.AL.: "A STUDY
ON BIOGLASS CERAMICS IN THE NA2O-
CAO-SIO2-P2O5 SYSTEM."**

(73) Titulaire : **FBFC INTERNATIONAL S.A.
24, rue de la Loi
B-1000 Bruxelles (BE)**

(72) Inventeur : **Ducheyne, Paul
27 Haymarket Lane
Bryn Nawar PA 9010 (US)**
Inventeur : **De Clercq, Marcel
Elzendreef 43
B-3110 Rotselaar (BE)**
Inventeur : **Van Hove, Louis
Leopoldlaan 10
B-2400 Mol (BE)**
Inventeur : **Schepers, Evert
Frans Vermylenstratt 16/6
B-3000 Leuven (BE)**
Inventeur : **Kempeneers, Raymond
25 Leopoldlaan
Mol (BE)**

(74) Mandataire : **Vanlaer, Marcel
PECHINEY 28, rue de Bonnel
F-69433 Lyon Cédex 3 (FR)**

## Description

## DOMAINE TECHNIQUE

L'invention concerne un matériau bioréactif, du type verre et son procédé de fabrication. Ce matériau est généralement destiné à être implanté, sous forme de composite, associé à un matériau de structure généralement à bas coéfficient de dilatation, dans le corps humain ou les animaux, par exemple en tant qu'implant dentaire ou prothèse osseuse.

## ETAT DE LA TECHNIQUE

Les prothèses osseuses et les implants sont réalisés à partir de matériaux tels que les métaux, céramiques, polymères, composites. Ils peuvent être différentiés par leur réactivité vis-à-vis du squelette et doivent présenter, outre une excellente biocompatibilité, de très bonnes caractéristiques mécaniques, qui, dans le cas des implants dentaires permet d'assurer une fonction masticatoire.

Les liaisons et/ou fixation avec les tissus osseux peuvent être réalisées de différentes façons: fixation mécanique, collage avec ciment organique ou minéral, ancrage biologique. Dans ce dernier cas la liaison peut se faire soit à l'aide d'une couche poreuse ménagée sur la prothèse, la repousse osseuse envahissant et comblant la porosité, soit à l'aide d'une liaison chimique entre une prothèse en matériau bioréactif et le tissu osseux (ancrage biologique par ostéoconductivité).

Dans le cadre de la présente invention on s'intéresse aux prothèses ou implants en matériau (à base de verre ou céramique) bioréactif donnant lieu à ancrage biologique par liaison chimique avec l'os. Les prothèses ou implants de même que les ancrages doivent supporter de fortes sollicitations mécaniques, notamment résistance à la rupture. C'est pourquoi l'invention se rapporte plus particulièrement aux prothèses ou implants, réalisés à partir desdits matériaux, renforcés par des fibres ou autres substrats (matériau de structure) ayant de bonnes caractéristiques mécaniques. En particulier on peut ainsi réaliser des implants dentaires comportant deux pièces: une racine implantée dans l'os de la mâchoire, sur laquelle sera ensuite fixée un passage permucosal qui recevra la prothèse dentaire externe.

Les matériaux à base d'hydroxyapatite $Ca_{10}(PO_4)_6(OH)_2$, de phosphate tricalcique $Ca_3(PO_4)_2$ sous ses formes $\alpha$ ou $\beta$, ou mieux les bioverres présentent une bioréactivité importante; leurs liaisons obtenues avec l'os entraînent un changement de composition de la couche superficielle de réaction située sur ledit matériau à l'interface avec l'os. Des expérimentations sur animaux ont montré que ladite couche superficielle de réaction présente des défauts de solidité mécanique sous charge du fait de la migration de la réaction vers l'intérieur du matériau. Ainsi pour éviter ces défauts la demanderesse a cherché à réduire l'épaisseur de ladite couche de réaction tout en préservant sa capacité de liaison avec les tissus osseux et a ainsi cherché à développer des matériaux (verres, céramiques, renforcés ou non) à bioréactivité réduite.

Ces matériaux bioréactifs peuvent être utilisés à la confection des prothèses ou implants composites contenant un matériau de structure en renfort comprenant par exemple un métal ou une céramique, ayant de ce fait des propriétés mécaniques améliorées. On réalise des composites plaqués ou armés. Des prothèses ou implants de bioverre armé de fibre d'acier inox, dans lesquels le bioverre et l'inox adhèrent bien l'un à l'autre, ont été expérimentés sur animaux. Mais ces implants armés d'inox présentent des inconvénients. En particulier, il est nécessaire que l'inox soit recouvert d'une gaine de verre, mais on observe après une certaine durée d'implantation que cette gaine peut se fissurer, l'inox ainsi mis à nu entre en contact avec le milieu biologique et il se produit autour de ces points des réactions inflammatoires inacceptables qui vont perturber l'ostéogénèse, empêcher une bonne liaison avec les tissus osseux et ainsi limiter la solidité de cette jonction. Cette mise à nu se rencontre également quand il y a des défauts (par exemple fissures) à la périphérie de la gaine de bioverre. Pour éviter une telle mise à nu de l'inox, l'usinage des prothèses ou implants est en particulier prohibé. On peut penser que ce phénomène inflammatoire est dû à l'action conjuguée de l'émission d'ions d'inox et d'ions de verre. Par ailleurs l'emploi de l'inox est à éviter du fait d'une biocompatibilité médiocre.

Ainsi la demanderesse a cherché à réaliser des composites de bioverre renforcés par des matériaux de structure ayant une bonne biocompatibilité comme certains métaux tels que Ti ou encore Nb, Ta ou commes des céramiques. De tels matériaux de structure peuvent ainsi être mis à nu et exposés au milieu biologique sans inconvénient lié à la biocompatibilité. Par ailleurs l'adhérence entre le matériaux bioréactif et le matériau de structure doit être la plus élevée possible. La demanderesse a constaté à ce sujet, que les hydroxyapatites, les phosphates tricalciques, ou les bioverres de l'art antérieur habituellement utilisés ont une adhérence encore imparfaite vis à vis des matériaux de structure envisagés par la demanderesse. Une mauvaise adhérence peut en effet entraîner un descellement du matériau de renfort, par exemple lors de l'application de la force masticatoire et entraîne en particulier une insuffisance des propriétés mécaniques du composite à renfort métallique obtenu.

De plus la demanderesse a cherché à mettre au point non seulement une liaison principale chimique améliorée entre le verre et le tissu osseux, mais aussi un agrippage secondaire mécanique, par croissance du tissu osseux dans les rugosités ou ondulations créées par la dissolution superficielle du matériau bioréactif, qui tend à améliorer la solidité mécanique de l'implantation.

Ces rugosités ou ondulations se produisent généralement dans un composite de matériau bioréactif armé où le matériau de structure affleure, après dissolution partielle dudit matériau bioréactif .

Ainsi le brevet US 4 478 904 (DUCHEYNE) décrit un composite armé constitué d'un squelette en fibre d'acier inox imprégné par trempage dans un bioverre fondu puis solidifié. Un tel composite présente l'inconvénient d'utiliser l'inox qui a une biocompatibilité médiocre et retarde l'ostéogénèse en conduisant aux inconvénients cités ci-dessus.

En particulier, dans le cas de cette association inox-bioverre, on note qu'il se forme une couche intermédiaire, entre le composite et le tissu ossifié formé, relativement épaisse (environ 200 à 300 μm) qui est relativement peu solide et se rompt lorsque l'implant est sollicité mécaniquement. A ce moment là de petites fissures peuvent se produire dans le bioverre de sorte qu'en ces endroits se forment des cloques de tissus non osseux qui perturbent la régénération du tissu osseux. Donc avec ce type de verres trop bioréactifs l'ancrage biologique est insuffisamment solide et la réaction biologique ne se limite pas à une couche de réaction superficielle, mais se propage à l'intérieur de la masse de verre entraînant sa fragilisation. Par ailleurs le type de verres décrit présentent une adhérence insuffisante sur les matériaux de renfort envisagés par la demanderesse.

La demande EP 0 206 726 (FLorida) décrit également le même type de formulation de verre ayant une bioréactivité importante, dans le but d'obtenir des granulés aptes à aider la réparation des cavités ou défauts osseux.

Le brevet US 4 775 646 (HENCH) décrit deux verres bioréactifs de compositions comprises dans les limites de composition des verres décrits dans les deux doucments précédents, mais dans lesquels une partie (30 à 60% molaire) de la chaux a été remplacée par $CaF_2$ dans le but de diminuer la réactivité du verre obtenu en vue d'augmenter sa résistance à la déminéralisation en milieu biologique. Ainsi ces verres contiennent au minimum 10 % en poids de $CaF_2$; une telle quantité de fluorure peut présenter des risques de toxicité. De plus on note qu'après fusion et solidification ces verres contiennent des espèces cristallisées (apatites...) (fig.2,6...).

Le document, Journal of Matérial Science (JMS) vol 23 (1988) page 4295-4299, décrit une composition de verre comportant, après fusion et solidification, des phases cristallines et présentant des caractéristiques mécaniques améliorées. Il ne comporte pas de fluorure.

## OBJET DE L'INVENTION

Face à ces problèmes la demanderesse a cherché, tout d'abord à mettre au point un matériau bioréactif qui, associé à un matériau de renfort (ou de structure), donne des pièces composites dotées d'une meilleure biocompatibilité. Parmi les matériaux de renfort envisagés on note en particulier le titane, mais aussi le niobium, le tantale, ou encore des céramiques commes les carbures, nitrures, borures, sous forme de poudre, de fibres, de trichites, de squelette poreux etc... qui en eux-mêmes présentent déjà une bonne biocompatibilité.

Elle a cherché également à disposer d'un matériau bioréactif, du type verre ou de préférence verre partiellement ou totalement cristallisé, compatible avec ces métaux ou matériaux dits de renfort ou de structure, c'est-à-dire :

. qui adhère mieux en particulier sur ledit matériau de structure de façon à obtenir soit des couches de placage, du matériau bioréactif sur le matériau de structure, qui ne glissent pas, soit des composites armés de meilleure qualité qui ne se délitent pas.

. qui ait un coefficient de dilatation très voisin, de préférence au plus égal à celui desdits matériaux de structure et particulièrement du Ti, de façon à supprimer toute apparition des fissures.

Elle a également cherché à disposer d'un matériau bioréactif doté encore d'une plus faible réactivité avec le tissu osseux de façon à obtenir une couche de réaction, avec le tissu osseux, mince tout en ayant une solidité améliorée capable de supporter des charges mécaniques importantes, et à diminuer la propagation de la réaction de dissolution lente (déminéralisation) dans la masse, qui tend à dégrader le bioverre, améliorant ainsi sa résistance à la rupture;

L'objectif est d'obtenir un ancrage biologique toujours plus solide et plus homogène, de façon à augmenter la durée de vie et la solidité de la prothèse ou de l'implant, de préférence amélioré par un agrippage secondaire mécanique obtenu par croissance du tissu osseux dans les rugosités ou ondulations pouvant se former à la surface du matériau bioréactif.

Elle a cherché encore à disposer de procédés d'obtention de pièces composites armées ou plaquées, à base dudit matériau bioréactif et desdits matériaux de renfort, préférant à l'utilisation d'une technique par verre fondu, celle d'un procédé contribuant à améliorer l'adhésion du matériau bioréactif et du matériau de structure. En effet le procédé d'imprégnation d'une préforme fibreuse par trempage dans du verre liquide décrit par exemple dans le brevet US 4 478 904 (DUCHEYNE) n'est pas applicable au Ti qui réagirait avec le verre aux hautes températures utilisées.

## DESCRIPTION DE L'INVENTION

L'invention est un verre bioréactif amélioré, de réactivité limitée, généralement mis en oeuvre en vue de l'obtention de pièces composites avec un matériau de structure à bas coéfficient de dilatation pour prothèse osseuse ou implant dentaire, ledit verre bioréactif se soudant par liaison chimique avec du tissu osseux, cette liaison chimique ne présentant pas de défaut d'ossification et ayant simultanément une faible épaisseur et une résistance mécanique très améliorée, ledit verre bioréactif ayant également un faible coefficient de dilatation et une très bonne adhérence sur ledit matériau de structure, caractérisé en ce qu'il contient, en % poids, 5 à 14% de $Na_2$, O, 0 à 12% de $P_2O_5$, 49 à 57% de $SiO_2$, le solde étant constitué d'au plus environ 33% d'un mélange de CaO et $CaF_2$, ce dernier étant compris entre 0,5 et 7 %.

Mais ce verre contient de préférence 8 à 12% de $Na_2O$, 4 à 8% de $P_2O_5$, 50 à 54% de $SiO_2$, la balance étant toujours d'au plus environ 33% et formée de CaO et $CaF_2$, ce dernier étant de préférence compris entre 0,5 et 5 %.

Le verre est de préférence partiellement cristallisé; le verre dans certains cas peut être totalement cristallisé. Ainsi un tel verre permet un ancrage biologique, par liaison ou soudure chimique avec le tissu osseux, particulièrment solide et résistant.

L'épaisseur de la couche de réaction dans ledit verre, c'est-à-dire la couche qui réagit avec le tissu osseux, est très faible tout en étant très solide. De plus la réaction qui est à l'origine de la liaison chimique ne se propage pas à l'intérieur du matériau bioréactif, évitant ainsi d'en altérer les caractéristiques mécaniques notamment sa résistance à la rupture; on a ainsi constaté qu'il est moins bioréactif que les verres connus décrits dans les documents cités ci-dessus, mais suffisamment pour assurer l'ancrage biologique et que la couche bioréactive peut supporter des charges ou efforts plus élevés. Les matériaux de structure de bonne biocompabilité envisagés pour obtenir des pièces composites sont généralement infusibles et inertes vis à vis du verre bioréactif, dans les conditions de préparation des composites qui seront décrites plus loin. Ils sont choisis parmi des métaux comme Ti et ses alliages, mais aussi Ta, Nb et leurs alliages, ou encore parmi des céramiques commes les carbures, borures, nitrures, les monocristaux tels que corindons, saphirs, diamants etc... On peut les employer sous forme de poudre, de fibres longues ou courtes (trichites) dispersées, de préformes fibreuses ou autres pièces de forme poreuse (obtenues par exemple par frittage de poudre), de pièces massives pleines ou creuses etc...

Pour obtenir une bonne adhérence du verre bioréactif sur le matériau de structure il est important que leur coefficient de dilatation soit voisin et que, de préférence, celui du verre bioréactif soit légèrement inférieur à celui du matériau de structure. On évite également de ce fait les risques de fissure et de fragilisation du matériau bioréactif notamment lors des phases de refroidissement se produisant au cours des procédés d'obtention. On note ainsi que les matériaux de structure ont un faible coéfficient de dilatation toujours inférieur à $10^{-5}$ $K^{-1}$ entre 0 et 100°C, alors qu'un acier inox a un coefficient de dilatation élevé voisin de $1,6.10^{-5}$ $K^{-1}$.

Ainsi le verre bioréactif selon l'invention possède un coéfficient de dilatation inférieur en général à $10^{-5}$ $K^{-1}$ et adhère parfaitement au matériau de structure, en particulier au Ti,(fibreux, poudreux...), sous forme massive ou divisée.

Les phases cristallines observées dans le produit final sont principalement la dévitrite $Na_2Ca_3Si_6O_{16}$ ou $(Na_2O)$ $(CaO)_3(SiO_2)_6$ mais également en quantité moindre la fluorapatite $Ca_{10}$ $(PO_4)_6F_2$ et l'agrellite $NaCa_2$ $Si_4O_{10}F$ ou $(NaF)(CaO)_2(SiO_2)_4$;
La présence d'alumine et/ou de composés de bore n'est pas souhaitable.

Le mélange précurseur contient les éléments cités ci-dessus dans les proportions requises; pour obtenir on introduit l'oxyde alcalin de préférence sous forme d'au moins l'un des composés du type carbonate, phosphate, phosphate acide..., le $P_2O_5$ de préférence sous forme d'un phosphate ou un phosphate acide alcalin ou alcalino terreux, par exemple $CaHPO_4$, $SiO_2$ sous forme telle quelle, l'oxyde alcalino terreux, en général CaO, de préférence sous forme de carbonate, phosphate, oxyde et le fluorure de préférence sous forme de $CaF_2$.

Les poudres sont dans des proportions telles que dans le produit final, les teneurs en cations, phosphate, et fluorure soient comprises dans les fourchettes décrites ci-dessus.

Ce mélange de poudre est ensuite fondu, homogénéisé, coulé; on laisse ensuite refroidir pour obtenir un verre non cristallisé. Ce verre peut être éventuellement recuit par exemple à 650°C pendant 4 h. Le verre intermédiaire ainsi obtenu est pratiquement exempt de phase cristalline et est ensuite broyé finement. La poudre obtenue a une composition homogène et est préférentiellement utilisée, dans une deuxième phase, pour la confection des composites, comme cela sera vu ultérieurement.

Le verre broyé peut être également, dans cette deuxième phase, soit mis en forme par pressage ou compactage à froid puis fritté naturellement ou fritté sous charge, soit directement fritté sous charge. Le frittage sous charge est toujours effectué après dégazage par mise sous vide; il est uni axe, multi-axe ou de préférence isostatique (HIP = Hot Isostatic Pressing). On obtient alors une pièce en verre bioréactif sous forme d'un verre pouvant contenir une phase cristallisée comme cela a été vu précédemment. On voit que cette deuxième phase du procédé, situé après l'obtention du verre intermédiaire broyé et conduisant au produit bioréactif final, se déroule généralement sans passer par l'état liquide.

Il est important que le verre intermédiaire obtenu après fusion de la poudre de départ puis solidification ne comporte pas de phase cristalline. En effet l'obtention des phases cristallisées souhaitées dans le matériau bioréactif final obtenu après frittage, et en particulier lors de la confection (selon le procédé qui sera vu plus loin) des composites armés (notamment ceux réalisés à partir d'un squelette de matériau de structure poreuse) ou plaqués de bonne qualité pourrait présenter des difficultés importantes.

Il est essentiel de maintenir la composition dans les limites prescrites étant donné que les différents éléments constitutifs sont imbriqués, ont des effets sur la structure du produit final et réagissent avec le milieu physiologique d'une façon optimale. Avec une telle combinaison on obtient de façon inattendue un ensemble de propriétés améliorant notamment à la fois l'ancrage biologique, les propriétés mécaniques du produit final, l'adhérence souhaitée sur des matériaux de structure en vue de l'obtention de pièces composites et une biocompatibilité et innocuité améliorées.

On peut ainsi noter que quand la teneur en oxyde alcalin exprimée en $Na_2O$, dans le produit final, est trop faible, outre que la fluidité du mélange fondu est insuffisante et qu'il est difficile d'obtenir un verre, la cristallisation dans le produit final est trop rapide et est mal contrôlée. En dessous de 5 % on n'obtient plus un verre non cristallisé, après fusion du mélange des poudres de départ puis refroidissement. La valeur minimum prescrite permet également d'amorcer la réaction assurant la liaison chimique avec le tissu osseux. Si au contraire on dépasse la limite supérieure décrite on ne peut plus obtenir de coefficent de dilatation suffisament bas, et on note une trop forte réactivité du verre avec les liquides physiologiques et donc un accroissement de la tendance à la désagrégation du matériau dans le temps et un affaiblissement préjudiciable des caractéristiques mécaniques de l'interface.

De même, la silice contribue à l'obtention d'une bioréactivité réduite: si sa teneur est supérieure aux limites décrites la bioréactivité devient trop faible, au détriment de la solidité de la liaison interfaciale avec l'os; l'ancrage biologique ne se réalise plus par exemple si la teneur en $SiO_2$ atteint 60 %. Si sa teneur est inférieure à la limite décrite; le coefficient de dilatation devient impropre et la bioréactivité trop importante, ce qui conduit à une couche de réaction épaisse et insuffisamment solide.

Quant à la présence obligatoire de fluorure elle est nécessaire pour initier et contrôler plus facilement la cristallisation lors de la deuxième phase du procédé. Elle permet également d'obtenir une bioréactivité réduite, de limiter la migration de la réaction de liaison, et ainsi de limiter la dissolution et les risques de désagrégation en profondeur du verre. Le fluorure entraîne la diminution de l'épaisseur de la couche de réaction jusqu'à la limite de détection, et l'amélioration de son homogénéité; de plus il donne au matériau une très grande stabilité à long terme en milieu biologique: dissolution négligeable et pas de fragilisation.

En cas de dépassement de la teneur prescrite en fluorure on note, compte tenu des faibles teneurs en $Na_2O$ et fortes teneurs en $SiO_2$ simultanément utilisées :

. une diminution de fluidité du verre fondu avec une probabilité de ne plus en obtenir;

. un déclenchement incontrôlé et néfaste de cristallisation dans le verre intermédiaire;

. un mauvais contrôle de cristallistion lors des traitements de frittage de la deuxième phase du procédé, ladite cristallisation devant se dérouler de préférence lentement, conduisant à des produits finaux (notamment composites) de qualité insuffisante;

. un étouffement progressif de la réaction d'ancrage biologique.

Par ailleurs, il est préférable de limiter le plus possible le fluorure pour diminuer les risques de toxicité liés à une présence trop importante. Cette limitation, comme cela a été déjà dit, est obtenue grâce à l'emploi en combinaison de quantités limitées de $Na_2O$ et plutôt importante de $SiO_2$ qui permettent par ailleurs une limitation de la réactivité du produit final et l'obtention de coefficient de dilatation compatible avec les composites visés.

Ainsi le verre bioréactif selon l'invention se distingue de celui décrit dans le brevet US 4 488 908 (DUCHEYNE), car il est adapté à l'obtention de composite comportant un matériau de structure à bas coefficient de dilatation.

Par ailleurs, il se distingue de ceux du brevet US 4 775 646 (HENCH) par les teneurs combinées en $Na_2O$, $SiO_2$ et fluorure; en effet dans (HENCH) les fortes teneurs en $Na_2O$ et les teneurs en $SiO_2$ favorables à l'obtention d'une forte réactivité sont compensées par de fortes teneurs en fluorure nécessaires pour diminuer suffisamment la réactivité, tandis que les coefficients de dilatation obtenus sont élevés. Ainsi avec de telles compositions, il semble difficile d'obtenir les faibles coefficients de dilatation souhaités dans l'invention et d'éviter les inconvénients dûs aux fortes teneurs en fluorure (toxicité, cristallisation...)

Enfin ce document ne permet pas de suggérer un produit dont la composition serait donnée par le document JMS dans lequel on aurait substitué CaO par $CaF_2$; en effet la teneur minimum en fluorure qui serait ainsi suggérée serait supérieure à la limite la plus élevée décrite dans l'invention.

Le verre selon l'invention est particulièrement adapté pour réaliser des prothèses osseuses ou des implants dentaires composites, c'est-à-dire qu'il est en général utilisé en association avec un autre matériau dit de structure qui sert à le renforcer. Ledit matériau de structure doit être infusible et inerte chimiquement dans les conditions d'élaboration du composite.

Les composites peuvent être du type armé ou plaqué.

Dans les pièces composites armées, les deux matériaux, verres bioréactifs et de structure, sont intimement liés; le matériau de structure peut alors se présenter au départ, soit sous forme de poudres ou de fibres dispersées, en vrac, mélangées aux poudres constituant le verre bioréactif, soit sous forme d'un squelette poreux (qui est une préforme, frittée ou non, formée de fibres enchevêtrées, ou une pré forme poreuse en matériau fritté) dans lequel sera introduit le matériau bioréactif. Le squelette présente généralement un volume poreux compris entre 15 et 90% et de préférence entre 30 et 70% du volume total, avant garnissage par le matériau bioréactif.

Dans les pièces composites plaquées, une pièce massive, creuse ou pleine, en matériau de structure est recouverte d'une couche fortement adhérente du verre bioréactif.

Le verre bioréactif selon l'invention convient particulièrement à la réalisation de pièces composites à base de Ti; une telle association se traduit par la quasi-disparition de la couche intermédiaire qui ne mesure plus que 0,02 à 2 μm et par la disparition complète des cloques de tissus non ossifié que l'on notait avec l'utilisation des composites bioverre-inox de l'art antérieur.

L'invention concerne également le procédé d'obtention de pièces composites contenant un matériau de structure tel que décrit plus haut. Il comporte les étapes suivantes:

a) on mélange des poudres de carbonate alcalin, silice, oxyde alcalino-terreux, phosphate et fluorure, dans les proportions voulues.

b) on fond ce mélange et on l'homogénéise, de préférence dans un récipient fermé de façon à limiter le départ de fluorure.

c) on coule pour fractionner la charge et on laisse refroidir. A ce stade, on obtient un verre selon l'invention qui peut être traité par recuit comme cela a déjà été dit.

d) on effectue un broyage fin du verre obtenu à l'étape c), pour obtenir une poudre non cristalline de granulométrie moyenne inférieure à environ 100 μm et de préférence à 40 μm, qui est éventuellement séchée.

e) on procède alors à l'obtention du composite à l'état cru, après avoir éventuellement conditionné et/ou préparé convenablement le matériau de structure. Plusieurs variantes peuvent alors être utilisées selon le type de pièces composites que l'on veut obtenir:

- pour un composite armé,

. soit on mélange de préférence à sec le matériau de structure sous forme de poudre ou de fibres dispersées à la poudre de matériau bioréactif broyée et séchée de l'étape d), ce mélange étant introduit dans un moule pour mise en forme

. soit on introduit dans un moule un squelette poreux ou une pièce massive recouverte d'un squelette poreux, en matériau de structure et on répartit régulièrement le verre broyé non cristallisé de l'étape d) dans le moule autour du squelette ou de la pièce. Il n'est pas exclu que. lorsqu'on répartit ledit verre broyé autour des pièces poreuses, il ne pénètre pas dans la porosité, ce qui présente un avantage.

- Pour un composite plaqué, on introduit dans un moule une pièce massive en matériau de structure et on répartit régulièrement la poudre de l'étape d) dans le moule autour de la pièce.

f) Une fois le moulage réalisé, on effectue :

- pour un composite armé, ne comportant pas de squelette poreux:

. soit un pressage à froid suivi d'un traitement thermique de frittage (ou cofrittage) naturel (solution 1) ou d un frittage sous charge (solution 2).

. soit directement un frittage sous charge (solution 3).

- pour un composite armé comportant un squelette poreux ou comportant une pièce massive recouverte d'un squelette poreux: la solution 2 et de préférence la solution 3.

- pour un composite plaqué: les solutions 1 ou 2 et de préférence la solution 3.

Les frittages sous charge sont effectuées après fermeture du moule et dégazage sous vide; ils peuvent être uniaxe, multi-axe, mais on préfère pratiquer un frittage isostatique (HIP).

Lors d'un frittage sous charge la pression doit être appliquée dès que le verre est suffisamment fluide et avant que la cristallisation ne réduise la fluidité du verre. En général on opère entre 500 et 850°C et de préférence entre 600°C et 800°C et à une température au moins supérieure à la température d'amolissement du verre et sous forte pression, cette dernière étant habituellement comprise entre 50 et 1000 bar. Ces conditions sont maintenues au moins une minute et de préférence au moins minutes, mais ne doivent pas être prolongées au-delà de 5 heures et de préférence de 2 heures pour éviter un grossissement néfaste des cristaux

g) on refroidit de préférence lentement, au moins dans la zone d'environ 500°C à l'ambiante.

h) un polissage ou usinage peut être effectué pour faire disparaître les éventuels défauts périphériques, tels que fissures superficielles..., le noyau de la pièce en étant pratiquement exempt. Pour les pièces composites armées comportant un squelette poreux en matériau de structure, et particulièrement en Ti, il est avantageux de poursuivre l'usinage jusqu'à ce que ledit matériau de structure affleure à la surface de la pièce composite.

Il est à noter que l'emploi du procédé HIP est particulièrement favorable à l'obtention d'une bonne adhérence entre le matériau bioréactif et le matériau de structure notamment lorsque c'est un métal et particulièrement le Ti.

Un tel procédé conduit généralement à un taux de cristallisation du verre bioréactif d'au moins 40% et plus généralement supérieur à 80% ledit taux peut aller dans certains cas jusqu'à la cristallisation totale. On obtient alors soit un composite cofritté contenant les grains ou les fibres de matériaux de structure dispersés dans le verre bioréactif, soit un composite plaqué dont le noyau en matériau de structure est recouvert d'une couche de verre bioréactif, soit un composite dont le matériau de structure est sous forme d'un squelette poreux, recouvrant ou non une pièce massive, dont le volume poreux est totalement occupé par le matériau bioréactif.

Dans le cas où la pièce composite est un implant dentaire, l'invention permet de réaliser un implant, en général cylindrique comportant un noyau massif recouvert d'un squelette poreux et percé d'une cavité coaxiale dans laquelle on pourra loger une tige (en vue d'y fixer un passage permucosal); on obtiendra ainsi une continuité métallique entre le squelette et le noyau.

Un tel procédé est particulièrement recommandé pour réaliser des composites à base de Ti qui ne peut pas être mis en contact, sans réagir, avec le verre bioréactif fondu; il se produit, selon le cas, soit un cofrittage de la poudre de verre et des fibres de Ti mélangées, soit un cofrittage du biomatériau sur la pièce massive de Ti avec éventuellement des incrustations en surface entre le métal et le verre bioréactif, soit une pénétration du biomatériau dans le squelette poreux.

Le procédé est également applicable aux autres matériaux de structure déjà cités. Mais dans la mesure où ces dits autres matériaux ne réagissent pas avec le verre bioréactif fondu, on peut remplacer les procédés de frittage par un trempage du squelette poreux dans le bain fondu, suivi d'un refroidissement et éventuellement d'un recuit vers 750°C pendant 4 h.

Il est souvent utile mais non nécessaire de préparer et/ou conditionner le matériau de structure (par exemple la poudre, les fibres, les squelettes poreux, les pièces massives...) avant de le mettre en contact avec la poudre précurseur du biomatériau.

Ces traitements qui peuvent être des décapages chimiques ou autres traitements de surface, permettent d'améliorer l'adhérence entre les matériaux.

Avec les matériaux de structure affleurant ou non à la surrface des composites selon l'invention, il ne se produit pas les phénomènes inflammatoires nocifs, lors de l'ostéogénèse, à l'interface os-composite observés avec les composites inox-verre de l'art antérieur. Il n'y a donc pas d'inconvénient à ce que ledit matériau de structure soit mis à nu et exposé au liquide biologique à la périphérie d'une prothèse, laquelle peut donc ête usinée sans conséquence inacceptable pour l'ostéogénèse.

L'adhérence entre le verre bioréactif et le matériau de structure est par ailleurs toujours excellente.

## EXEMPLES

L'exemple 1 illustrera les problèmes rencontrés avec des implants composites confectionnés selon l'art antérieur; l'exemple 2 illustrera la confection d'implants composites selon l'invention et les résultats obtenus, après implantation chez l'animal.

## EXEMPLE.1

On a réalisé des implants dentaires selon l'art antérieur en utilisant de l'acier inox et un verre du type 45-S-5 contenant en poids:

$SiO_2$ 45%
$Na_2O$ 24,5%
CaO 24,5%
$P_2O_5$ 6%

Ils sont constitués d'une pièce cylindrique creuse fermée à une extrémité, ladite pièce étant un composite de fibre d'acier inox imprégné de verre bioréactif 45-S-5, obtenu par trempage des fibres dans le verre fondu. La pièce est recouverte extérieurement d'une gaine dudit verre bioréactif, sauf à son extrémité restée ouverte. Dans la cavité de la pièce cylindrique est fixée sans jeu une pièce massive en acier inox à l'extrémité supérieure de laquelle peut être fixé un passage permucosal en inox, assurant la liaison entre l'ensemble précédent appelé racine et la cavité buccale, et permettant l'installation de la prothèse dentaire.

La présence d'une gaine de verre bioréactif autour de la racine est nécessaire pour éviter un contact direct, entre les tissus osseux et l'acier inox du composite, qui empêcherait l'ostéogénèse et défavoriserait ainsi irrémédiablement un bon ancrage de l'implant.

Les implants ont été installés dans la mâchoire partiellement édentée de plusieurs chiens.

Après trois mois d'implantation, la moitié d'entre eux ont été réséqués et analysés microscopiquement et microchimiquement.

Des fissures ont été observées dans la gaine de verre par lesquelles les fibres d'acier inox sont en contact avec le liquide physiologique et on observe alors une inhibition locale de l'ostéogénèse par action simultanée des ions inox et des ions verre; les fissures peuvent elles-mêmes conduire à des inflammations.

L'autre moitié, après installation du passage permucosal, a été soumise à l'application des forces masticatoires. La majorité des implants se sont montrés défectueux sous charge à cause d'un affaiblissement de la liaison entre la gaine de verre bioréactif et les tissus osseux, la fragilisation étant également due à la présence à la surface extérieure de la gaine de verre d'une couche de réaction trop épaisse, quelques centaines de μm. En effet le verre étant trop réactif, la réaction chimique s'est propagée aisément dans la gaine et l'on a noté des changements de composition du verre qui le fragilisent.

## EXEMPLE 2

Il relate illustrativement la confection d'un implant dentaire composite selon l'invention et les résultats après implantation sur l'animal.
On a tout d'abord confectionné un squelette en fibres de Ti.
Pour cela on a utilisé des fibres en vrac de longueur 4 mm et de diamètre environ 50 μm. La longueur et le diamètre sont ajustables selon la taille des pièces à produire et peuvent être beaucoup plus importants dans le cas, par exemple où on voudrait confectionner des prothèses osseuses de dimensions plus importantes.
Il est préférable de leur faire subir un traitement de conditionnement, qui dans ce cas a été le suivant :
on les désagglomère sur tamis vibrant de maille grossière ;
on les décape à l'aide d'une solution aqueuse contenant 20% d'acide nitrique et 2% d'acide fluorhydrique ;
on lave, on sèche puis on effectue une deuxième opération de tamisage ;
on précompacte et on désagrège.

On compacte alors à froid directement à la forme et à la dimension finale voulue, compte tenu du retrait prévisible lors du frittage et du remplissage du volume poreux, pour obtenir un squelette cylindrique poreux (taux de vide 60%), et on élimine les fibres non agglomérées.
On fritte sous vide à 1150°C pendant 2 Heures.
On usine un trou dans l'axe du cylindre et on y ajuste ensuite une pièce en Ti massif (un tel montage est prévu pour monter par la suite un passage permucosal nécessaire à la mise en place de la prothèse dentaire).
Après un décapage alcalin, on effectue un deuxième frittage destiné à lier le Ti massif et le squelette fibreux.

On effectue ensuite de préférence une anodisation, qui améliorera l'adhérence de la céramique bioréactive, à l'aide d'un électrolyte contenant $H_3PO_4$ - $H_2SO_4$ - $Na_2HPO_4$ - $SiO_2,xH_2O$.
On prépare par ailleurs un mélange de poudre contenant (en poids) :

| | |
|---|---|
| $SiO_2$ | 41,0% |
| $Na_2CO_3$ | 13,5% |
| $CaCO_3$ | 33,9% |
| $CaHPO_4$ | 9,1% |
| $CaF_2$ | 2,5% |

que l'on introduit dans un creuset couvert.
On fond ce mélange à 1350°C, on l'homogénéise, et on coule pour fractionner la charge.

Après refroidissement on recuit à 650°C durant 4 h et on laisse refroidir en 20 h.
On broye et on obtient une granulométrie moyenne de 15 μm.
On sèche la poudre.
On introduit ledit squelette de Ti dans un moule en cuivre et la poudre séchée tout autour, et on ferme le moule sous vide par soudage.
On effectue un frittage isostatique en portant la température à 800°C, à raison de 30°C par minute, et en appliquant à ce moment une pression variant régulièrement de 200 à 1000 bar, et en maintenant ces conditions pendant 1 h.
On laisse refroidir lentement.

Après démoulage, on polit le composite obtenu et on l'amène à sa forme finale en faisant apparaître les fibres de Ti qui affleurent en surface; la répartition surfacique de Ti est homogène et le rapport de la surface des fibres apparentes à la surface du matériau bioréactif est voisin de $\frac{65}{35}$

Les résultats obtenus sont les suivants:
La teneur finale en F est de 0,92% (en poids)
L'implant tel quel est installé comme précédemment dans la mâchoire de chiens.

Après 3 mois d'implantation on a fait les observations suivantes:
- l'interface entre le composite titane-verre bioréactif et le tissu osseux ne montre pas de défaut d'hétérogénéité comme précédemment; l'ostéogénèse n'y est pas perturbée bien que les fibres métalliques soient exposées au liquide physiologique.
- la couche de réaction est homogène et a une épaisseur extrêmement réduite (quelques microns); de même les changements dans la composition du matériau bioréactif sont très peu étendus en profondeur et ne fragilisent pas le verre. La liaison osseuse et les caractéristiques ostéconductives du matériau bioréactif sont bien maintenues. Ainsi ledit matériau reste intact et garde sa solidité.

Il s'ensuit qu'on n'a pas noté de défectuosité dans la tenue mécanique de la liaison osseuse, ni de fissures ou autres défauts dans le verre et l'implant.
- la liaison osseuse avec le matériau bioréactif est déjà presque complète. Il semble que l'os formé aille d'une plage de matériau bioréactif à une autre en formant un petit pont de tissus osseux enjambant les fibres de Ti apparentes. Dans la zone intermédiaire située entre les fibres de Ti et le pont osseux on trouve parfois du tissu conjonctif, au contact du Ti, surmonté d'ostéoïde. Cette zone intermédiaire se transformera donc en tissus osseux ce qui assurera un contact direct entre ledit tissu osseux et le Ti.

## Revendications

1. Verre bioréactif amélioré de réactivité limitée, de préférence au moins partiellement cristallisé, généralement mis en oeuvre en vue de l'obtention de pièces composites avec un matériau de structure à bas coefficient de dilatation pour prothèse osseuse ou implant dentaire, ledit verre bioréactif se soudant par liaison chimique avec un tissu osseux, cette liaison ne présentant pas de défauts d'ossification et ayant simultanément une faible épaisseur et une résistance mécanique très améliorée, ledit verre bioréactif ayant également un faible coefficient de dilatation de dilatation et une très bonne adhérence sur ledit matériau de structure, caractérisé en ce qu'il contient, en % poids, 5 à 14% de $Na_2O$, 0 à 12% de $P_2O_5$, 49 à 57% de $SiO_2$, le solde étant constitué d'au plus 33% d'un mélange de CaO et $CaF_2$ ce dernier étant compris entre 0,5 et 7%.

2. Verre selon la revendication 1 caractérisé en ce qu'il contient 8 à 12% de $Na_2O$, 4 à 8% de $P_2O_5$, 50 à 54% de $SiO_2$, le solde étant d'au plus 33% d'un mélange de CaO et $CaF_2$ et contenant de 0,5 à 5% de $CaF_2$.

3. Verre selon l'une quelconque des revendications 1 et 2 caractérisé en ce qu'il a un taux de cristallisation final d'au moins 40%, de préférence supérieur à 80%.

4. Procédé de fabrication du verre de l'une quelconque des revendications 1 à 3 caractérisé en ce qu'on part d'un mélange de poudres, précurseur dudit verre de carbonates, de phosphates, de silice, d'oxydes et de fluorures, que l'on fond, homogénéise, coule, refroidit pour obtenir un verre intermédiaire non cristallisé, que l'on recuit éventuellement, puis broie, le verre non cristallisé broyé obtenu étant ensuite soit pressé à froid pour mise en forme puis fritté naturellement ou fritté sous charge après dégazage, soit fritté sous charge directement après dégazage, puis refroidi et éventuellement poli ou usiné.

5. Procédé de fabrication du verre des revendications 1 à 3 caractérisé en ce que on part d'un mélange de poudres, précurseur dudit verre bioréactif, de carbonate alcalin, de phosphate, de silice, d'oxyde alcalino-terreux et de fluorure, que l'on fond, homogénéise, coule, refroidit pour obtenir un verre non cristallisé, que l'on recuit éventuellement, qui est ensuite broyé, mis en présence du matériau de structure, l'ensemble verre non cristallisé, broyé et matériau de structure étant ensuite soit pressé à froid pour mise en forme, puis fritté naturellement ou fritté sous charge après dégazage, soit fritté sous charge directement après dégazage, puis refroidi et éventuellement poli ou usiné.

6. Procédé selon l'une quelconque des revendications 4 ou 5 caractérisé en ce que le frittage sous charge est une compaction isostatique à chaud (HIP = Hot Isostatic Pressing).

7. Composite contenant le verre bioréactif des revendications 1 à 3 et un matériau de structure.

8. Composite selon la revendication 7 caractérisé en ce qu'il est un composite armé dans lequel ledit verre bioréactif et ledit matériau de structure sont intimément liés.

9. Composite selon la revendication 7 caractérisé en ce qu'il est un composite plaqué dans lequel ledit verre bioréactif revêt ledit matériau de structure, ce dernier étant sous forme d'une pièce massive creuse ou pleine.

10. Composite selon l'une quelconque des revendications 7 à 9 caractérisé en ce que ledit matériau de structure est le Ti ou ses alliages.

11. Composite selon l'une quelconque des revendications 7 à 9 caractérisé en ce que ledit matériau de structure est choisi parmi Ta, Nb ou leurs alliages.

12. Composite selon l'une quelconque des revendications 7 à 9 caractérisé en ce que ledit matériau de structure est choisi parmi les fibres céramique ou métallique, les carbures, borures, nitrures, les monocristaux.

13. Composite selon l'une quelconque des revendications 7,8,10,11,12 caractérisé en ce que le matériau de structure est sous forme de grains ou de fibres dispersés dans le verre bioréactif.

14. Composite selon l'une quelconque des revendications 7,8,10,11,12 caractérisé en ce que le matériau de structure est sous forme de squelette poreux dont le volume poreux est occupé par le verre bioréactif.

15. Composite selon la revendication 14 caractérisé en ce que ledit squelette a un volume poreux compris entre 15 et 90% et de préférence entre 30 et 70% du volume total avant occupation par le verre bioréactif.

16. Composite selon l'une quelconque des revendications 14-15 caractérisé en ce que le squelette poreux est une préforme fibreuse ou un matériau fritté.

17. Composite selon l'une quelconque des revendications 7 à 16 caractérisé en ce qu'il sert à confectionner des prothèses osseuses humaines ou animales et de préférence des implants dentaires.

18. Procédé d'obtention selon l'une quelconque des revendications 5 ou 6 de pièces composites plaquées de l'une quelconque des revendications 7,9,10,11,12 caractérisé en ce que la mise en présence consiste à introduire dans un moule une pièce massive en matériau de structure, et à répartir le verre non cristallisé broyé régulièrement autour de la pièce massive.

19. Procédé d'obtention selon l'une quelconque des revendications 5 ou 6 de pièces composites armées de l'une quelconque des revendications 7,8,10,11,12,13 caractérisé en ce que la mise en présence consiste à introduire dans un moule, pour mise en forme, un mélange du verre non cristallisé broyé, et de poudre ou de fibres dispersées du matériau de structure, éventuellement préparées et conditionnées, pour obtenir "in fine" un composite cofritté.

20. Procédé d'obtention selon l'une quelconque des revendications 5 ou 6 de pièces composites armées de l'une quelconque des revendications 7,8,10,11,12,14,15,16 caractérisé en ce que la mise en présence consiste à introduire dans un moule un squelette poreux, ou une pièce massive recouverte d'un squelette poreux, en matériau de structure, et à répartir régulièrement le verre broyé non cristallisé dans le moule, autour dudit squelette ou de ladite pièce massive.

21. Procédé selon l'une quelconque des revendications 18,19,20 caractérisé en ce que la granulométrie moyenne du verre broyé, est inférieure à 100 µm et de préférence inférieure à 40 µm.

22. Procédé selon l'une quelconque des revendications 18 à 21 caractérisé en ce que le frittage sous charge est effectué entre 500 et 850°C et de préférence 600 et 800°C et à une température au moins supérieure à la température d'amolissement du verre, la pression étant comprise entre 50 et 1000 bar, ces conditions étant maintenues au moins une minute, et de préférence au moins 15 minutes et au plus 5 heures et de préférence 2 heures.

23. Procédé selon l'une quelconque des revendications 20 à 22 caractérisé en ce que l'on utilise un squelette poreux en fibres de Ti, préparé en partant de fibres en vrac qui subissent successivement les opérations suivantes : désagglomération par tamisage, décapage de préférence à l'aide d'une solution d'acide nitrique contenant de l'acide fluorhydrique, recuit, deuxième tamisage après refroidissement, précompactage, désagglomération, compactage à froid à la forme voulue, frittage sous vide, décapage avec une solution alcaline, de préférence anodisation, avant mise en place dudit squelette poreux conditionné dans le moule.

24. Procédé selon l'une quelconque des revendication 18, 20 à 23 caractérisé en ce que le polissage ou l'usinage fait affleurer le matériau de structure en surface de la pièce composite.

## Claims

1. An improved bioreactive glass of limited reactivity, preferably at least partly crystallised, which is usually used in order to produce composite pieces with a structural material which has a low expansion coefficient for a bone prosthesis or a dental implant, said bioreactive glass being welded by chemical bonding to a bone tissue, the bonding being free from ossification flaws and simultaneously being of low thickness and being much improved in terms of its mechanical strength, said bioreactive glass also having a low expansion coefficient and very good adherence to said structural material, characterised in that it contains, in % by weight, 5 to 14% $Na_2O$, 0 to 12% $P_2O_5$, 49 to 57% $SiO_2$, the balance being constituted of a maximum of 33% of a mixture of CaO and $CaF_2$, this latter being between 0.5 and 7%.

2. A glass according to Claim 1, characterised in that it contains 8 to 12% $Na_2O$, 4 to 8% $P_2O_5$, 50 to 54% $SiO_2$, the balance being a maximum of 33% of a mixture of CaO and $CaF_2$ and containing 0.5 to 5% $CaF_2$.

3. A glass according to any one of Claims 1 and 2, characterised in that it has a final crystallisation rate of at least 40%, preferably greater than 80%.

4. A process for the production of glass according to any one of Claims 1 to 3, characterised in that the process starts with a mixture of powders, a forerunner of said glass made of carbonates, phosphates, silica, oxides and fluorides which is melted, rendered homogeneous, cast, cooled to obtain a non-crystallised intermediate glass which may be annealed and then ground, the ground non-crystallised glass obtained then being either pressed cold for shaping and then fritted naturally or being fritted under a load after degassing, or being fritted under a load directly after degassing, and then cooled and possibly polished or machined.

5. A process for the production of glass according to Claims 1 to 3, characterised in that the process starts with a mixture of powders, a forerunner of said bioreactive glass, made of alkali metal carbonate, phosphate, silica, alkaline earth metal oxide and fluoride which is melted, rendered homogeneous, cast, cooled to obtain a non-crystallised glass which may be annealed and which is then ground, placed in the presence of the structural material, the unit of non-crystallised ground glass and the structural material then either being pressed cold for shaping, then being fritted naturally or fritted under a load after degassing, or being fritted under a load directly after degassing, then cooled and possibly polished or machined.

6. A process according to any one of Claims 4 or 5, characterised in that the fritting operation under a load is hot isostatic pressing.

7. A composite containing bioreactive glass according to Claims 1 to 3 and a structural material.

8. A composite according to Claim 7, characterised in that it is a reinforced composite in which said bioreactive glass and said structural material are closely bonded.

9. A composite according to Claim 7, characterised in that it is a plated composite in which said bioreactive glass covers said structural material, this latter being in the form of a hollow or filled solid piece.

10. A composite according to any one of Claims 7 to 9, characterised in that said structural material is Ti or its alloys.

11. A composite according to any one of Claims 7 to 9, characterised in that said structural material is selected from Ta, Nb or their alloys.

12. A composite according to any one of Claims 7 to 9, characterised in that said structural material is selected from ceramic or metallic fibres, carbides, borides, nitrides, monocrystals.

13. A composite according to any one of Claims 7, 8, 10, 11, 12, characterised in that the structural material is in the form of grains or fibres which are dispersed in the bioreactive glass.

14. A composite according to any one of Claims 7, 8, 10, 11, 12, characterised in that the structural material is in the form of a porous skeleton whose pore volume is occupied by the bioreactive glass.

15. A composite according to Claim 14, characterised in that said skeleton has a pore volume of between 15 and 90%, and preferably between 30 and 70%, of the total volume prior to being occupied by the bioreactive glass.

16. A composite according to any one of Claims 14-15, characterised in that the porous skeleton is a fibrous preform or a fritted material.

17. A composite according to any one of Claims 7 to 16, characterised in that it is used to make human or animal bone prostheses, and preferably dental implants.

18. A method according to any one of Claims 5 or 6 for obtaining plated composite pieces according to any one of Claims 7, 9, 10, 11, 12, characterised in that the placing in the presence operation consists in introducing a solid piece of structural material into the mould, and in spreading the ground non-crystallised glass uniformly around the solid piece.

19. A method according to any one of Claims 5 or 6 for obtaining reinforced composite pieces according to any one of Claims 7, 8, 10, 11, 12, 13, characterised in that the placing in the presence operation consists in introducing into a mould for shaping purposes a mixture of ground non-crystallised glass and dispersed powder or fibres of the structural material, possibly prepared and conditioned, so that a co-fritted composite is finally obtained.

20. A method according to any one of Claims 5 or 6 for obtaining reinforced composite pieces according to any one of Claims 7, 8, 10, 11, 12, 14, 15, 16, characterised in that the placing in presence operation consists in introducing into a mould a porous skeleton or a solid piece covered with a porous skeleton, made of structural material, and in uniformly spreading the non-crystallised ground glass in the mould, around said skeleton or said solid piece.

21. A method according to any one of Claims 18, 19, 20, characterised in that the mean grain size of the ground glass is less than 100 $\mu$m and preferably less than 40 $\mu$m.

22. A process according to any one of Claims 18 to 21, characterised in that the fritting operation under a load is carried out between 500 and 850°C and preferably between 600 and 800°C and at a temperature which is at least higher than the softening temperature of the glass, the pressure being between 50 and 1000 bar, these conditions being kept for at least one minute, and preferably for at least 15 minutes and for a maximum of 5 hours and preferably 2 hours.

23. A process according to any one of Claims 20 to 22, characterised in that a porous skeleton is used which is made of Ti fibres and prepared from bulk fibres which are successively subjected to the following operations: deagglomeration by screening, pickling preferably by the use of a nitric acid solution containing hydrofluoric acid, annealing, a second screening process after cooling, precompacting, deagglomeration, compacting cold into the desired shape, fritting under vacuum, pickling with an alkaline solution, preferably anodisation, with said conditioned porous skeleton being placed in position in the mould.

24. A process according to any one of Claims 18, 20 to 23, characterised in that the polishing or machining operation makes the structural material level with the surface of the composite piece.

**Patentansprüche**

1. Verbessertes, bevorzugt teilweise kristallisiertes bioreaktives Glas von begrenzter Reaktivität, das im allgemeinen im Hinblick auf die Herstellung von Verbundwerkstoffen mit einem Grundmaterial von geringem Ausdehnungskoeffizienten für Knochenprothesen oder Zahnimplantate eingesetzt wird, wobei das bioreaktive Glas durch Bildung chemischer Bindungen mit einem Knochengewebe zusammenwächst, wobei die entstehende Verbindungsschicht keine Mängel bei der Knochenbildung mit sich bringt und gleichzeitig eine geringe Dicke und eine stark verbesserte mechanische Festigkeit hat, und das ferner einen geringen Ausdehnungskoeffizienten aufweist und gut auf dem Grundmaterial haftet, dadurch **gekennzeichnet**, daß es 5 bis 14 Gew.-% $Na_2O$, 0 bis 12 Gew.-% $P_2O_5$ und 49 bis 57 Gew.-% $SiO_2$ enthält und der Rest von höchstens 33 Gew.-% aus einem Gemisch von CaO und $CaF_2$ besteht, worin $CaF_2$ mit 0,5 bis 7 Gew.-% enthalten ist.

2. Glas nach Anspruch 1, dadurch gekennzeichnet, daß es 8 bis 12 Gew.-% $Na_2O$, 4 bis 8 Gew.-% $P_2O_5$ und 50 bis 54 Gew.-% $SiO_2$ enthält und der Rest von höchstens 33 Gew.-% aus einem Gemisch von CaO und $CaF_2$ mit 0,5 bis 5 Gew.-% $CaF_2$ besteht.

3. Glas nach Anspruch 1 oder 2, dadurch gekennzeichnet, daß es einen End-Kristallinitätsgrad von mindestens 40 % und bevorzugt mehr als 80 % aufweist.

4. Verfahren zur Herstellung des Glases nach einem der Ansprüche 1 bis 3, gekennzeichnet durch folgende Schritte: Verwendung eines Pulvergemischs als Vorstufe des bioreaktiven Glases aus Carbonaten, Phosphaten, Siliciumdioxid, Oxiden und Fluoriden, Schmelzen, Homogenisieren, Gießen und Abkühlen unter Erhalt eines nichtkristallinen Zwischenglases, gegebenenfalls erneutes Erhitzen und danach Zerkleinern des nichtkristallinen Zwischenglases und anschließend entweder Kaltpressen des erhaltenen nichtkristallinen Glases zur Formgebung und Sintern unter Atmosphärendruck oder Sintern unter Druck nach Entgasen oder Sintern unter Druck unmittelbar nach dem Entgasen und Abkühlen und gegebenenfalls Polieren oder Bearbeitung.

5. Verfahren zur Herstellung des Glases nach den Ansprüchen 1 bis 3, gekennzeichnet durch folgende Schritte: Verwendung eines Pulvergemischs als Vorstufe des bioreaktiven Glases aus Alkalicarbonat, Phosphat, Siliciumdioxid, Erdalkalioxid und Fluorid, Schmelzen, Homogenisieren, Gießen und Abkühlen unter Erhalt eines nichtkristallinen Glases, gegebenenfalls erneutes Erhitzen und danach Zerkleinern und Zusammenbringen mit dem Grundmaterial und anschließend entweder Kaltpressen der Einheit aus dem zerkleinerten nichtkristallinen Glas und dem Grundmaterial zur Formgebung und Sintern unter Atmosphärendruck oder Sintern unter Druck nach Entgasen oder Sintern unter Druck unmittelbar nach dem Entgasen, Abkühlen und gegebenenfalls Polieren oder Bearbeitung.

6. Verfahren nach Anspruch 4 oder 5,
dadurch gekennzeichnet, daß
es sich beim Sintern unter Druck um isostatisches Heißpressen handelt (HIP = Hot Isostatic Pressing).

7. Verbundwerkstoffe, die ein bioreaktives Glas nach einem der Ansprüche 1 bis 3 und ein Grundmaterial enthalten.

8. Verbundwerkstoffe nach Anspruch 7,
dadurch gekennzeichnet, daß
es sich um armierte Verbundwerkstoffe handelt, in denen das bioreaktive Glas und das Grundmaterial eng verbunden sind.

9. Verbundwerkstoffe nach Anspruch 7,
dadurch gekennzeichnet, daß
es sich um plattierte Verbundwerkstoffe handelt, bei denen das bioreaktive Glas als Überzug auf dem Grundmaterial vorliegt, das einen Hohlkörper oder einen massiven Körper darstellt.

10. Verbundwerkstoffe nach einem der Ansprüche 7 bis 9,
dadurch gekennzeichnet, daß
das Grundmaterial Titan oder eine seiner Legierungen ist.

11. Verbundwerkstoffe nach einem der Ansprüche 7 bis 9,
dadurch gekennzeichnet, daß
das Grundmaterial unter Ta, Nb und deren Legierungen ausgewählt ist.

12. Verbundwerkstoffe nach einem der Ansprüche 7 bis 9,
dadurch gekennzeichnet, daß
das Grundmaterial unter Keramikfasern, metallischen Fasern, Carbiden, Boriden, Nitriden und Einkristallen ausgewählt ist.

13. Verbundwerkstoffe nach einem der Ansprüche 7, 8, 10, 11 und 12,
dadurch gekennzeichnet, daß
das Grundmaterial in Form von Körnern oder Fasern vorliegt, die im bioreaktiven Glas dispergiert sind.

14. Verbundwerkstoffe nach einem der Ansprüche 7, 8, 10, 11 und 12,
dadurch gekennzeichnet, daß
das Grundmaterial in Form eines porösen Gerüsts vorliegt, dessen Porenvolumen mit dem bioreaktiven Glas ausgefüllt ist.

15. Verbundwerkstoffe nach Anspruch 14,
dadurch gekennzeichnet, daß
das Gerüst ein Porenvolumen von 15 bis 90 Vol.-% und bevorzugt von 30 bis 70 Vol.-% besitzt, bezogen auf das Gesamtvolumen vor dem Ausfüllen mit dem bioreaktiven Glas.

16. Verbundwerkstoffe nach Anspruch 14 oder 15,
dadurch gekennzeichnet, daß
das poröse Gerüst ein Faservorformkörper oder ein gesintertes Material ist.

17. Verbundwerkstoffe nach einem der Ansprüche 7 bis 16,
dadurch gekennzeichnet, daß
sie zur Anfertigung von Knochenprothesen für Menschen oder Tiere und bevorzugt zur Anfertigung von Zahnimplantaten dienen.

18. Verfahren nach Anspruch 5 oder 6 zur Herstellung plattierter Verbundwerkstoffteile nach einem der Ansprüche 7, 9, 10, 11 und 12,
dadurch gekennzeichnet, daß
das Zusammenbringen der Komponenten darin besteht, daß ein massives, aus dem Grundmaterial bestehendes Teil in eine Gußform eingesetzt und das zerkleinerte nichtkristalline Glas gleichmäßig um dieses massive Teil herum verteilt wird.

19. Verfahren nach Anspruch 5 oder 6 zur Herstellung von armierten Verbundwerkstoffteilen nach einem der Ansprüche 7, 8, 10, 11, 12 und 13,
dadurch gekennzeichnet, daß
das Zusammenbringen der Komponenten darin besteht, daß zur Formgebung ein Gemisch aus dem zerkleinerten nichtkristallinen Glas und darin dispergiertem Pulver oder Fasern des Grundmaterials, die gegebenenfalls vor- und aufbereitet wurden, zum Erhalt von am Ende zusammengesinterten Verbundwerkstoffen in eine Gußform eingefüllt wird.

20. Verfahren nach Anspruch 5 oder 6 zur Herstellung von armierten Verbundwerkstoffteilen nach einem der Ansprüche 7, 8, 10, 11, 12, 14, 15 und 16, dadurch gekennzeichnet, daß
das Zusammenbringen der Komponenten darin besteht, daß ein poröses Gerüst aus dem Grundmaterial oder ein massiver Körper, der von einem porösen Gerüst aus dem Grundmaterial bedeckt ist, in eine Gußform eingesetzt wird und das zerkleinerte nichtkristalline Glas gleichmäßig in der Gußform um das Gerüst oder den massiven Körper verteilt wird.

21. Verfahren nach einem der Ansprüche 18, 19 und 20,

dadurch gekennzeichnet, daß
die mittlere Korngröße des gemahlenen Glases kleiner als 100 μm und vorzugsweise kleiner als 40 μm ist.

22. Verfahren nach einem der Ansprüche 18 bis 21, dadurch gekennzeichnet, daß
die Sinterung unter Druck bei 500 bis 850°C und vorzugsweise bei 600 bis 800°C sowie einer Temperatur, die zumindest höher ist als die Erweichungstemperatur des Glases, unter einem Druck von 50 bis 1000 bar durchgeführt wird, wobei diese Bedingungen mindestens 1 min und vorzugsweise mindestens 15 min und höchstens 5 h und vorzugsweise 2 Stunden aufrechterhalten werden.

23. Verfahren nach einem der Ansprüche 20 bis 22, dadurch gekennzeichnet, daß
ein poröses Gerüst aus Titanfasern verwendet wird, das aus losen Fasern hergestellt ist und das nacheinander folgenden Verfahrensschritten unterzogen wurde:
Entfernung von Agglomeraten durch Sieben, Beizen, bevorzugt mit einer Salpetersäurelösung, die Fluorwasserstoffsäure enthält, Erwärmen, zweites Sieben nach Abkühlen, Vorverdichten, Entfernung von Agglomeraten, Kaltverdichten zur gewünschten Form, Sintern im Vakuum, Beizen mit einer alkalischen Lösung und bevorzugt anodische Oxidation vor dem Einsetzen des konditionierten porösen Gerüsts in die Gußform.

24. Verfahren nach einem der Ansprüche 18 und 20 bis 23,
dadurch gekennzeichnet, daß
das Polieren oder die Bearbeitung dazu führt, daß das Grundmaterial aus der Oberfläche des Verbundwerkstoffteils ein wenig herausragt.